# EUROPEAN PATENT SPECIFICATION

(11) **EP 2 023 995 B1**
(45) Date of publication and mention of the grant of the patent: **17.02.2016**
(21) Application number: 07789579.5
(22) Date of filing: 22.05.2007
(51) Int. Cl.: A61M 25/00, A61F 2/04

(54) **PROSTATIC STENT**
PROSTATASTENT
STENT PROSTATIQUE

(30) Priority: 22.05.2006 US 802112 P
(43) Date of publication of application: 18.02.2009
(73) Proprietor: Devonec, Marian, 01700 Miribel (FR)
(72) Inventor: Devonec, Marian, 01700 Miribel (FR)
(74) Representative: Chevalier, Renaud Philippe
(86) International application number: PCT/IB2007/002172
(87) International publication number: WO 2007/135564

(56) References cited:
- WO-A-03/011179
- WO-A-03/070310
- DE-U1- 9 213 656
- US-A- 3 938 529
- US-A1- 2001 041 883
- US-A1- 2003 040 803
- US-A1- 2004 087 886
- US-A1- 2004 143 209
- US-A1- 2006 079 835

## Description

The invention concerns a prostatic stent, in particular a prostatic stent able to be used in combination with an introducer in order to treat bladder outlet obstruction, as generally described in WO 2006126060.

A prostatic stent known from the prior art is disclosed in WO 91/00712. Such prostatic stent comprises a flexible tubular member having a lateral wall **and the bladder side of the stent** equipped with anchoring means, adapted for anchoring the prostatic stent into the urethra.

The stent is able, due to the flexibility of the tubular member, to accommodate the natural anatomical bend of the prostatic urethra.

The anchoring means are twofold:
- the first anchoring means is formed by a roughened outer jacket, mounted on the tubular element. The outer jacket comprises a plurality of sharp elements extending outwardly or inwardly, thereby providing anchoring into the urethra.

However, such anchoring means generally cause damage to the urethra during insertion or withdrawal of the stent. Indeed, the mucous membrane can be torn by the sharp anchoring means. In this case, blood clot can appear, thereby obstructing the lumen of the stent and the urethra.
- the second anchoring means is a circular flange located on the bladder side of the stent. The flange in contact with the bladder (neck) orifice has an outer diameter larger than the diameter of the bladder orifice and thus prevents the stent from downward migration.

Other prostatic stents are disclosed in US 2003/0040803 A1 and in US 2001/0041883 A1.

With this concept the length of the stent must be adapted to the exact length of the prostatic urethra. A given prostate will be best stented by a stent with the appropriate length: too short a stent may not release the obstruction, too long it crosses through the urinary sphincter resulting in patient's incontinence.

For this reason a set of stents with lengths increasing by increments of 5 to 10 mm must be available with this concept. The length of the obstructed urethra has to be measured by endoscopy or ultrasonography to choose among the set of stents ranging from 30 up to 80 mm length, the stent with the appropriate length for a given prostate.

The invention as defined in claim 1 intends to avoid such drawbacks by proposing a prostatic stent offering a sufficient anchoring into the prostatic urethra without damaging it during its insertion or removal and a prostatic stent with a single length allowing the catheterization of the majority of prostates enlarged by benign prostatic hypertrophy.

To this end, the invention concerns a prostatic stent of the above mentioned type, characterized in that said anchoring means comprise a succession of projecting and hollow elements defining a continuous and smooth anchoring profile, thereby forming an anchor for the prostatic stent in place into the urethra, and adapted to avoid any lesion of the urethra during insertion or withdrawal of the stent.

After insertion of the stent, the mucous membrane tends to conform the wavy profile of the stent so that the membrane penetrates the hollow elements and that the projecting elements cooperate with the membrane to enhance the anchoring.

The anchoring means comprise an helical groove.

Such helical groove allows the stent to be sufficiently flexible and also to be rigid enough in order to be easily inserted into the urethra. Such groove also provides a low pressure contact with the lumen minimizing prostatic urethra irritation. The groove also facilitates an angulation of the tubular member by more than 90 degrees without closure of the lumen of the tubular member.

Preferably, the tubular member comprises a distal end closed by a closing member, the lateral wall comprising at least one aperture enabling the urine from the bladder to flow through the tubular member.

The closed distal end provides an abutment for the distal end of a stylet. The use of a stylet is well know from the prior art.

According to one embodiment of the invention, the tubular member and the closing member are made out of one piece.

According to another embodiment of the invention, the tubular member and the closing member are two distinct parts, the closing member being fixedly engaged on the tubular member, notably by welding or by over moulding.

Preferably, the tubular member comprises an opened distal end.

According to another embodiment of the invention, the proximal end of the tubular member is equipped with flexible tongues, each of said tongues being arranged to be elastically biased toward a radial and outward position.

The flexible tongues are forming an half-Malecot part which is able to be deployed at the level of the veru montanum in the prostatic portion of the urethra. The tongues, due to their specific orientation and their flexibility, tend to flatten during insertion of the stent whereas they spread at the level of the veru montanum as soon as the stent has been realeased. In the released state, the tongues prevent the stent from downward migration. Mild traction on the thread attached to the proximal end of the stent results in the abutment of the tongues above the striated sphincter, which means correct positioning.

According to one particular embodiment of the invention, the lateral wall is equipped with at least one multi-winged Malecot.

The Malecot also prevent the stent from downward migration. In this case, mild traction on the thread attached to the proximal end of the stent results also in the flattening of the wings of the Malecot. Thus abutment against the sphincter is not perceptible.

Preferably, the length of the stent is in the range of 80 mm.

Such length allows catheterization of the prostatic urethra of the majority of patients using this single or unique dimension. Indeed, the dimensions of the prostate may differ from one patient to another. It is important that the distal end of the stent is located into the bladder. However, the length of the part of the stent which projects from the prostatic urethra into the bladder does not have to be taken into account by the physician because any patient will easily tolerate a 10 to 50 mm projecting part of the stent inside the bladder.

Fifty millimetres is the length which will protrude inside the bladder of a patient with a normal size prostate, but with a bladder orifice sclerosis. A 50 mm length is the common length of Foley catheter protruding inside the bladder (diameter of the inflated balloon plus tip of the Foley catheter).

The physician is therefore not obliged to use a set of stents of different lengths and to measure the length of the prostatic urethra in order to determine which stent could be the most adapted to the patient's prostate.

According to the invention the tubular member is made of silicone.

In another embodiment of the invention, the length of the stent is in the range of 80 mm.

Preferably, the tubular element comprises a portion able to elongate along its longitudinal axis.

It is therefore possible to adjust the length of the stent with regard to the size of the biggest prostates.

The present invention is now described with reference to annexed drawing wherein:
Figure 1 a is a front elevation of a first embodiment of the stent according to the invention, the stent comprising an helical groove ;
Figure 1b shows a stent comprising accordion pleated grooves ;
Figure 2a is a view of the stent in a bent position;
Figure 2b is a partly cross-section view of the stent in a bent position;
Figure 3 represents the stent linked to an additional tubular element.
Figures 4 to 7 represent other embodiments of the invention.

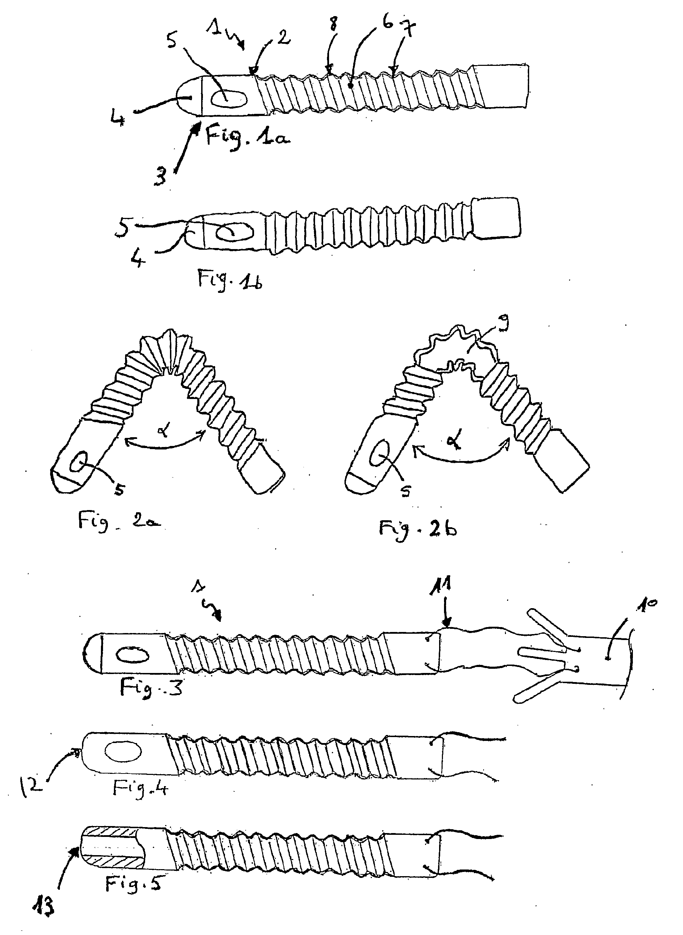

As shown in Figure 1, the invention concerns a prostatic stent comprising a tubular member 1 made of silicone and having a lateral wall 2.

The inner diameter of the tubular member allows the insertion of a stylet (as shown and described in WO 2006126060) whereas the outer diameter allows the catheterization of most adult patient with a normal urethra, without urethral stricture. It must be understood that the insertion of such a stent can be managed by the way of most of the wellknown methods, for example as described and claimed in WO 2006126060.

The length of the stent is in the range of 80 mm.

The tubular member 1 comprises a distal part 3 which is cylindrical, the distal end being closed by a closing member 4.

The closing member 4 is fixedly engaged on the tubular member, notably by welding or by over moulding.

The lateral wall 2 comprises two opposite apertures 5 enabling the urine from the bladder to be drained through the tubular member 1.

The tubular member further comprises a median part 6 which is equipped with anchoring means, adapted for anchoring the prostatic stent into the obstructed prostatic urethra.

Said anchoring means comprise an helical grove 7 (Figure 1 a) arranged in form of a screw thread or accordion pleated groves (Figure 1 b) with rounded crests 8, defining a continuous and smooth anchoring with a sinusoidal profile.

Such smooth anchoring profile forms an anchor for the prostatic stent when the stent is in place into the urethra, and is also adapted to avoid any lesion of the urethra during insertion or withdrawal of the stent.

Figure 2 represents the stent in a bended state, where the tubular member 1 is bent with an angle α less than 90 degrees (Figure 2a). As it can be seen from figure 2b, the lumen 9 formed by the tubular part in the bent region is not closed due to the flexibly of the tubular member 1. In addition, due to the elasticity of the tubular member 1, the latter recovers its original form after bending. This facilitates retrieval of the stent.

As detailed on figure 3, the tubular member 1 is able to be linked to an additional tubular member 10 in a manner similar to the known prior art (see for example WO 2006126060) through flexible connecting means 11.

The additional tubular member 10 comprises flanges forming an half Malecot, able to abut against the urinary striated sphincter and to provide a clear tactile feed back to the physician, thereby allowing correct positioning of the stent only by hand without the need of any external equipment to control the positioning.

In another embodiment of the invention detailed on figure 4, the tubular member 1 and the closing member 4 are made out of one piece. The tubular element then comprises a blind tip 12 of the Foley type.

In a still other embodiment detailed on figure 5, the tubular member 1 comprises an opened distal end 13. In such case, there is no need to provide additional apertures in the lateral wall 2.

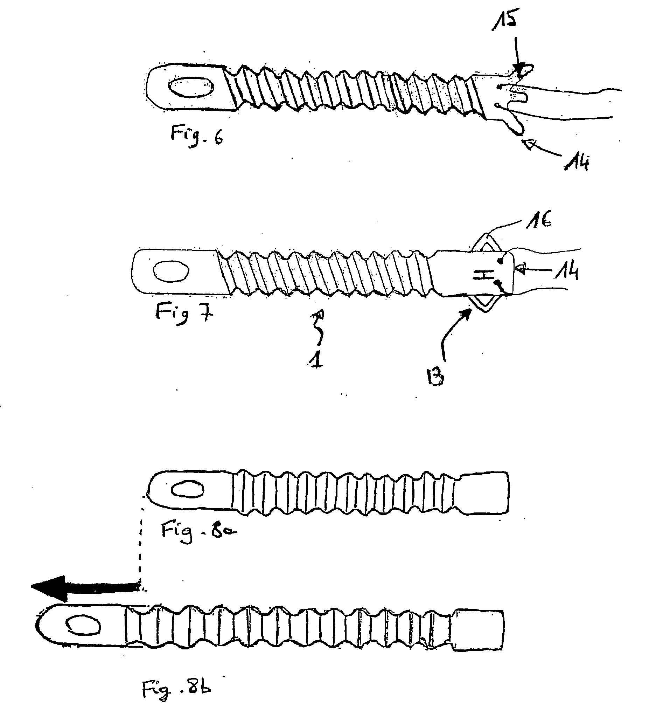

In another embodiment detailed on figure 6, the proximal end 14 of the tubular member is equipped with flexible tongues 15, each of said tongues being arranged to be elastically biased toward a radial and outward position, thereby forming an half Malecot.

In a still other embodiment detailed on figure 7, the lateral wall 2 is equipped with a multi-winged Malecot 16. The multi-winged Malecot 16 is located on the proximal end 14 of the tubular element 1.

In figure 8, the accordion pleated wall (Figure 8a) can be stretched longitudinally to increase the length of the stent by 10 to 20 mm (Figure 8b) in the case of stenting a huge size prostate.

It is obvious that the features of the embodiments detailed on figure 3 can be combined with the features of the embodiments detailed on figures 4 to 7.

## Claims

1. A prostatic stent comprising a flexible tubular member (1) having a lateral wall (2) equipped with anchoring means, adapted for anchoring the prostatic stent into the prostatic urethra, said anchoring means comprise a succession of hollow and projecting elements (7, 8) defining a continuous and smooth anchoring profile, thereby forming an anchor for the prostatic stent in place into the prostatic urethra, and adapted to avoid any lesion of the urethra during insertion or withdrawal of the stent,
**characterized in that**:
- the anchoring means comprise an helical groove (7) which allows the prostatic stent to be sufficiently flexible and also rigid enough in order to be easily inserted into the urethra so that in a bended state of the prostatic stent (1), where the tubular member (1) is bent with an angle α less than 90 degrees, a lumen (9) formed by the tubular member (1) in a bent region is not closed,
- the tubular member is made of silicone.

2. A prostatic stent according to claim 1, **characterized in that** the tubular member (1) comprises a distal end (3) closed by a closing member (4), the lateral wall (2) comprising at least one aperture (5) enabling the urine from the bladder to flow through the tubular member (1).

3. A prostatic stent according to claim 2, **characterized in that** the tubular member (1) and the closing member (4) are made out of one piece.

4. A prostatic stent according to claim 2, **characterized in that** the tubular member (1) and the closing member (4) are two distinct parts, the closing member (4) being fixedly engaged on the tubular member (1), notably by welding or by over moulding.

5. A prostatic stent according to one of the claims 1 to 4, **characterized in that** the tubular member (1) comprises an opened distal end (13).

6. A prostatic stent according to one of the claims 1 to 5, **characterized in that** the proximal end (14) of the tubular member (1) is equipped with flexible tongues (15), each of said tongues (15) being arranged to be elastically biased toward a radial and outward position.

7. A prostatic stent according to one of the claims 1 to 5, **characterized in that** the lateral wall (2) is equipped with at least one multi-winged Malecot (16).

8. A prostatic stent according to one of the claims 1 to 7, **characterized in that** the length of the stent is in the range of 80 mm.

9. A prostatic stent according to one of the claims 1 to 8, **characterized in that** the tubular element comprises a portion able to elongate along its longitudinal axis.

## Patentansprüche

1. Prostatastent aus einem flexiblen rohrförmigen Element (1) mit einer Seitenwand (2), ausgestattet mit Verankerungsmitteln, die ausgelegt sind, um den Prostatastent in der prostatischen Urethra zu verankern, wobei die Verankerungsmittel eine Aufeinanderfolge von vertieften und vorstehenden Elementen (7, 8) umfassen, die ein kontinuierliches und glattes Verankerungsprofil definieren, wodurch ein Anker für den Prostatastent in Position in der prostatischen Urethra gebildet wird, und ausgelegt, um jede Verletzung der Urethra während des Einführens oder der Entnahme des Stents zu vermeiden,
**dadurch gekennzeichnet, dass**
- die Verankerungsmittel eine schraubenförmige Nut (7) umfassen, die ermöglicht, dass der Prostatastent ausreichend flexibel und auch starr genug ist, um einfach in die Urethra eingeführt zu werden, so dass in einem gebogenen Zustand des Prostatastents (1), in dem das rohrförmige Element (1) mit einem Winkel α von weniger als 90 Grad gebogen ist, ein Lumen (9), gebildet durch das rohrförmige Element (1) in einem gebogenen Bereich, nicht geschlossen ist,
- das rohrförmige Element aus Silikon hergestellt ist.

2. Prostatastent nach Anspruch 1, **dadurch gekennzeichnet, dass** das rohrförmige Element (1) ein distales Ende (3) umfasst, das durch ein Verschlusselement (4) geschlossen ist, wobei die Seitenwand (2) mindestens eine Öffnung (5) umfasst, die ermöglicht, dass der Harn aus der Blase durch das rohrförmige Element (1) fließt.

3. Prostatastent nach Anspruch 2, **dadurch gekennzeichnet, dass** das rohrförmige Element (1) und das Verschlusselement (4) aus einem Stück hergestellt sind.

4. Prostatastent nach Anspruch 2, **dadurch gekennzeichnet, dass** das rohrförmige Element (1) und das Verschlusselement (4) zwei verschiedene Teile sind, wobei das Verschlusselement (4) fest auf dem rohrförmigen Element (1) eingegriffen ist, insbesondere durch Schweißen oder Umspritzen.

5. Prostatastent nach einem der Ansprüche 1 bis 4, **dadurch gekennzeichnet, dass** das rohrförmige Element (1) ein geöffnetes distales Ende (13) umfasst,

6. Prostatastent nach einem der Ansprüche 1 bis 5, **dadurch gekennzeichnet, dass** das proximale Ende (14) des rohrförmigen Elements (1) mit flexiblen Zungen (15) ausgestattet ist, wobei jede der Zungen (15) angeordnet ist, um elastisch hin zu einer radialen und äußeren Position vorgespannt zu sein.

7. Prostatastent nach einem der Ansprüche 1 bis 5, **dadurch gekennzeichnet, dass** die Seitenwand (2) mit mindestens einem mehrflügligen Malecot (16) ausgestattet ist.

8. Prostatastent nach einem der Ansprüche 1 bis 7, **dadurch gekennzeichnet, dass** die Länge des Stents im Bereich von 80 mm liegt.

9. Prostatastent nach einem der Ansprüche 1 bis 8, **dadurch gekennzeichnet, dass** das rohrförmige Element einen Abschnitt umfasst, der dazu in der Lage ist, sich entlang seiner Längsachse zu verlängern.

## Revendications

1. Stent prostatique comprenant un élément tubulaire flexible (1) ayant une paroi latérale (2) équipée de moyens d'ancrage, adaptés pour ancrer le stent prostatique dans l'urètre prostatique, lesdits moyens d'ancrage comprennent une succession d'éléments creux et en saillie (7, 8) définissant un profil d'ancrage continu et lisse, formant ainsi un ancrage pour le stent prostatique en place dans l'urètre prostatique, et adaptés pour éviter toute lésion de l'urètre lors de l'insertion ou du retrait du stent,
**caractérisé en ce que** :
- les moyens d'ancrage comprennent une rainure hélicoïdale (7) qui permet au stent prostatique d'être suffisamment flexible et aussi assez rigide afin d'être facilement inséré dans l'urètre de sorte que dans un état courbé du stent prostatique (1), dans lequel l'élément tubulaire (1) est courbé selon un angle α inférieur à 90°, une lumière (9) formée par l'élément tubulaire (1) dans une zone courbée ne soit pas fermée,
- l'élément tubulaire est réalisé en silicone.

2. Stent prostatique selon la revendication 1, **caractérisé en ce que** l'élément tubulaire (1) comprend une extrémité distale (3) fermée par un élément de fermeture (4), la paroi latérale (2) comprenant au moins une ouverture (5) permettant à l'urine provenant de la vessie de s'écouler à travers l'élément tubulaire (1).

3. Stent prostatique selon la revendication 2, **caractérisé en ce que** l'élément tubulaire (1) et l'élément de fermeture (4) sont réalisés en une seule pièce.

4. Stent prostatique selon la revendication 2, **caractérisé en ce que** l'élément tubulaire (1) et l'élément de fermeture (4) sont deux parties distinctes, l'élément de fermeture (4) étant en prise fixe sur l'élément tubulaire (1), notamment par soudage ou par surmoulage.

5. Stent prostatique selon l'une des revendications 1 à 4, **caractérisé en ce que** l'élément tubulaire (1) comprend une extrémité distale ouverte (13).

6. Stent prostatique selon l'une des revendications 1 à 5, **caractérisé en ce que** l'extrémité proximale (14) de l'élément tubulaire (1) est équipée de languettes flexibles (15), chacune desdites languettes (15) étant agencée pour être sollicitée de manière élastique vers une position radiale et extérieure.

7. Stent prostatique selon l'une des revendications 1 à 5, **caractérisé en ce que** la paroi latérale (2) est équipée d'au moins un dispositif de Malécot à plusieurs branches (16).

8. Stent prostatique selon l'une des revendications 1 à 7, **caractérisé en ce que** la longueur du stent est de l'ordre de 80 mm.

9. Stent prostatique selon l'une des revendications 1 à 8, **caractérisé en ce que** l'élément tubulaire comprend une partie capable de s'allonger le long de son axe longitudinal.
